# EUROPEAN PATENT APPLICATION

(11) **EP 1 489 103 A1**
(43) Date of publication of application: **22.12.2004**
(21) Application number: 03712688.5
(22) Date of filing: 13.03.2003
(51) Int. Cl.: C08F 4/52, A61K 6/00, A61K 6/083, A61L 24/00

(54) **PASTE POLYMERIZATION INITIATOR COMPOSITION, DENTAL OR SURGICAL ADHESIVE AND ADHESIVE KIT**

(30) Priority: 28.03.2002 JP 2002092992
(71) Applicant: SUN MEDICAL CO., LTD., Shiga-ken 524-0044 (JP)
(72) Inventor: TOMIKAWA, Tamotsu, c/o SUN MEDICAL CO., LTD, Moriyama-shi, Shiga 524-0044 (JP); ZENG, Weiping, c/o SUN MEDICAL CO., LTD, Moriyama-shi, Shiga 524-0044 (JP)
(74) Representative: HOFFMANN - EITLE
(86) International application number: PCT/JP2003/003020
(87) International publication number: WO 2003/082931

(57) **Abstract**

The present invention is a polymerization initiator paste composition which comprises (A) an organoboron compound which is liquid at 25°C and (B) particles having an average particle diameter of 0.001 to 50 µm and inert to the organoboron compound (A), in an amount of 4 to 400 parts by weight based on 100 parts by weight of the organoboron compound (A), and has a consistency, as measured at 25°C under a load of 300 g in accordance with a method defined in ISO4823, of 15 to 100 mm. This composition constitutes a dental kit or the like together with a polymerizable monomer separately packaged. According to the present invention, an organoboron compound can be used safely, and because the composition is a paste, it is very easily utilized.

## Description

### TECHNICAL FIELD

The present invention relates to a polymerization initiator paste composition that is favorably used as a polymerization initiator of a dental or surgical adhesive, a dental or surgical adhesive using the paste composition, and a kit of the adhesive. More particularly, the invention relates to a polymerization initiator paste composition that is excellent in storage stability, handling properties and economical efficiency, a dental or surgical adhesive showing excellent adhesion to dentin and hard tissue, and a dental or surgical adhesive kit.

### BACKGROUND ART

Organoboron compounds, particularly tributylboron (TBB.) or TBB partial oxide, have been used for years as polymerization initiators of dental adhesives because of their excellent adhesion properties to dentin.

TBB, however, is a low-viscosity liquid at a working temperature (room temperature) and reacts with oxygen in air at room temperature. This reaction is an exothermic reaction, and if TBB that is in contact with a combustible substance such as paper reacts with oxygen, ignition (firing) of the substance sometimes takes place. On this account, in order to slightly decrease reactivity of TBB to facilitate handling of TBB, a part of TBB is allowed to react with oxygen previously to form a partial oxide of TBB, and the partial oxide is often employed. However, even if the partial oxide of TBB comes into contact with oxygen, the reaction proceeds. Therefore, TBB or its partial oxide is filled in a special syringe container having airtight properties, so that improvement of handling properties of TBB and simplification of the container have been desired.

Some attempts to inhibit fuming- or firing-causing properties of organoboron compounds have been heretofore made.

In Japanese Patent Publication No. 37092/1976, there is disclosed a dental or surgical adhesive using a product (partially oxidized trialkylboron), as a polymerization initiator, obtained by the reaction of trialkylboron, with 0.3 to 0.9 mol of oxygen and decreased in the activity.

In Japanese Patent Laid-Open Publication No. 11892/1973, there is disclosed a method to improve safety against fuming by the use of a paste obtained by adding a substance having hydrophobicity and viscosity, such as vaseline, paraffin or silicone (silicone oil), and if necessary, an adsorbing agent, such as silicic acid or alumina, to trialkylboron or its derivative.

In Japanese Patent Publication No. 54683/1991 (U.S. Patent No. 4,676,858, European Patent No. 78994), there is proposed a polymerization initiator that is a homogeneous mixture obtained by adding an organic oligomer or an organic polymer, such as silicone oil, wax, oligoester or oligoamide, to an organoboron compound.

In Japanese Patent Laid-Open Publication No. 264509/1991, there is proposed a method to improve safety by the use of a paste obtained by adding a polymerization product of a (meth)acrylic acid alkyl ester having a particle diameter of 1 to 100 µm to tributylboron or partially oxidized tributylboron.

In Japanese Patent Laid-Open Publication No. 110913/1997, there is described a composition obtained by mixing an organoboron compound with an inert low-boiling solvent and a polymer or an oligomer.

In Japanese Patent Laid-Open Publication No. 95806/2000, it is described that a specific oligoester is added as a stabilizer to an organoboron compound and an organic peroxide is added to a polymerizable reactive component to optimize the time to reach the final strength and the adhesion strength and that an initiator component is enclosed in a plastic ampule made of a laminate of a metal foil and a synthetic material of low oxygen diffusion properties to form a kit.

With regard to TBB, various improvements have been proposed as disclosed in the above publications, but in any of those proposals, there is yet room for further improvement in the enhancement of handling properties of TBB and the simplification of containers.

### DISCLOSURE OF THE INVENTION

It is an object of the present invention to provide a polymerization initiator paste composition which is a paste or a cream containing a liquid organoboron compound and causing no burning or firing of paper even if it is brought into contact with paper in air, hardly shows stringiness and is activated by oxygen in air to impart high polymerizability to a polymerizable composition and thereby cure it for a short period of time.

It is another object of the present invention to provide a polymerization initiator composition which hardly shows stringiness, has been improved in the handling properties and the container cost by being contained in a general purpose container such as an aluminum tube capable of accurately measuring out the composition even if the amount used one time is small, e.g., several mg to several ten mg, and is particularly favorable for a dental or surgical adhesive.

It is a further object of the present invention to provide a dental or surgical adhesive and an adhesive kit each of which uses the above-mentioned polymerization initiator composition of the invention and exhibits high adhesion properties.

The polymerization initiator paste composition of the present invention comprises:
(A) an organoboron compound which is liquid at 25°C, and
(B) particles having an average particle diameter of 0.001 to 50 µm and inert to the organoboron compound (A), in an amount of 4 to 400 parts by weight based on 100 parts by weight of the organoboron compound (A), and
   has a consistency, as measured at 25°C under a load of 300 g in accordance with a method defined in ISO4823, of 15 to 100 mm.

The dental or surgical adhesive of the present invention comprises:
a polymerization initiator paste composition comprising:
   (A) an organoboron compound which is liquid at 25°C, and
   (B) particles having an average particle diameter of 0.001 to 50 µm and inert to the organoboron compound (A), in an amount of 4 to 400 parts by weight based on 100 parts by weight of the organoboron compound (A), and
      having a consistency, as measured at 25°C under a load of 300 g in accordance with a method defined in ISO4823, of 15 to 100 mm,
      and
   (C) a polymerizable monomer which is packaged separately from the polymerization initiator paste composition.

The dental or surgical adhesive kit of the present invention comprises:
a first container filled with a polymerization initiator paste composition comprising:
   (A) an organoboron compound which is liquid at 25°C, and
   (B) particles having an average particle diameter of 0.001 to 50 µm and inert to the organoboron compound (A), in an amount of 4 to 400 parts by weight based on 100 parts by weight of the organoboron compound (A) ,
having a consistency, as measured at 25°C under a load of 300 g in accordance with a method defined in ISO4823, of 15 to 100 mm, and
being extrudable from the container,
and
a second container filled with a polymerizable monomer (C) which is contained separately from the polymerization initiator paste composition.

According to the present invention, by adding a specific powder (B) to an organoboron compound (A), the organoboron compound (A) that is liquid at room temperature is changed to a semisolid having a specific consistency. The organoboron compound (A) in a semisolid state having a specific consistency is filled in a tube or a syringe, and the semisolid is extruded in a necessary amount (necessary length) every time it is used, whereby the organoboron compound can be quantitatively determined and used. Further, because of the given consistency, the paste organoboron compound (polymerization initiator paste composition) squeezed (or extruded) retains its extruded shape and dose not run out. The thus extruded paste can be easily mixed with a polymerizable polymer.

Moreover, the amount of the organoboron compound can be converted into a length of the extruded paste, and therefore, the organoboron compound can be quantitatively determined and used without performing a complicated operation such as metering.

### BRIEF DESCRIPTION OF THE DRAWINGS

Fig. 1 is a sectional view of an example of a container filled with a polymerization initiator paste composition of the present invention.
Fig. 2 is a sectional view of another example of a container filled with a polymerization initiator paste composition of the present invention.
Fig. 3 is a sectional view of a further example of a container filled with a polymerization initiator paste composition of the present invention.

### BEST MODE FOR CARRYING OUT THE INVENTION

The polymerization initiator paste composition, the dental or surgical adhesive and the adhesive kit according to the present invention are described in detail hereinafter.

The polymerization initiator paste composition of the invention comprises (A) an oraganoboron compound and (B) particles.

The organoboron compound (A) for use in the invention is preferably liquid at room temperature (25°C), and is a polymerization initiator capable of initiating polymerization of a compound having unsaturated group such as a (meth)acrylic acid ester. Preferred examples of the organoboron compounds include trialkylboron, alkoxyalkylboron, dialkylborane and partially oxidized trialkylboron. These compounds can be used singly or in combination.

The trialkylboron is, for example, triethylboron, tripropylboron, triisopropylboron, tributylboron, tri-sec-butylboron, triisobutylboron, tripentylboron, trihexylboron, trioctylboron, tridecylboron, tridodecylboron, tricyclopentylboron or tricyclohexylboron.

The alkoxyalkylboron is, for example, butoxydibutylboron.

The dialkylborane is, for example, 9-borabicyclo[3.3.1]nonane.

The partially oxidized trialkylboron is, for example, partially oxidized tributylboron. As the partially oxidized trialkylboron, a trialkylboron partial oxide obtained by the addition of preferably 0.3 to 0.9 mol, more preferably 0.4 to 0.6 mol, of oxygen to 1 mol of trialkylboron is available.

Of the above liquid organoboron compounds, trialkylboron or partially oxidized trialkylboron is particularly preferably employed. Most preferable as the liquid organoboron compound is tributylboron (TBB) or partially oxidized tributylboron.

As the organoboron compound (A) employable in the invention, a compound which is not liquid at room temperature (25°C) but can be dissolved or dispersed in a solvent to form a solution or a dispersion is also available. An example of such an organoboron compound is a phenyl borate compound.

Examples of the phenyl borate compounds include tetraphenylboron alkali metal salt, tetraphenylboron triethanolamine salt, tetraphenylboron dimethyl-p-toluidine salt, tetraphenylboron ethyl dimethylaminobenzoate, sodium tetrakis(p-fluorophenyl)boron, tetraphenylboron triethylamine salt, tetraphenylboron dimethylaminoethyl methacrylate salt and sodium butyltri(p-fluorophenyl)boron.

The particles (B) for use in the invention are particles which are inert to the organoboron compound (A), are solid at 25°C and have an average particle diameter of 0.001 to 50 µm. These particles (B) can be dissolved or homogeneously dispersed in the organoboron compound (A) to adjust a consistency of the organoboron compound to such a degree that the organoboron compound can be extruded quantitatively from, for example, a tubular container. A semisolid of such a consistency is generally called a paste, a jelly or a cream, but in the present invention, they are sometimes generically referred to as a "paste".

In the present invention, it is desirable to use, as the particles (B), particles which are solid at room temperature (25°C), preferably at 37°C. When such particles are used for a curing agent of a dental or surgical adhesive, there is an advantage that retention of adhesion strength can be enhanced.

In the present invention, particles which are inert to the organoboron compound (A) and are solid at 25°C are used as the particles (B). The particles are, for example, polymer particles which do not contain a group having active hydrogen, such as a hydroxyl group or an amino group, and/or inorganic particles. As the particles (B), polymer particles and/or inorganic particles having an average particle diameter of 0..001 to 50 µm are preferably used. More preferably used as the particles (B) are polymer particles having an average particle diameter of 0.001 to 30 µm and/or inorganic particles having an average particle diameter of 0.001 to 10 µm, and particularly preferably used are polymer particles and/or inorganic particles having an average particle diameter of not less than 0.005 µm and less than 1 µm.

In the case where the polymer particles are used as the particles (B) in the invention, preferred examples of polymers to form the polymer particles include synthetic polymers, such as poly(meth)acrylic acid esters, polystyrene, butadiene polymers (e.g., MBS), polyacrylonitrile, polyamide (Nylon™), silicone resin, polyurethane, melamine resin, phenolic resin and fluororesin; and natural polymers, such as cellulose, cellulose acetate and chitosan. Of these, poly(meth)acrylic acid esters are particularly preferably employed. In the present invention, the particles (B) have only to be solid at room temperature, and instead of the polymer particles, oligomer particles may be employed. The polymer or the oligomer to form the polymer particles has a molecular weight of usually 1000 to 1000000. Although the polymer particles may be spherical or in unfixed shapes, they are preferably spherical.

In the case where the poly(meth)acrylic acid ester particles are used as the particles (B) in the invention, examples of (meth)acrylic acid ester monomers used to form the particles include alkyl acrylates, such as methyl acrylate, ethyl acrylate, propyl acrylate, isopropyl acrylate, butyl acrylate, sec-butyl acrylate, isobutyl acrylate, t-butyl acrylate, pentyl acrylate and hexyl acrylate; and alkyl methacrylates, such as methyl methacrylate, ethyl methacrylate, propyl methacrylate, isopropyl methacrylate, butyl methacrylate, sec-butyl methacrylate, isobutyl methacrylate, t-butyl methacrylate, pentyl methacrylate and hexyl methacrylate. These monomers can be used singly or in combination. As the polymer particles, particles prepared by usual polymerization can be used as they are, or their pulvarizates can be used.

In the case where the polymer particles insoluble in the organoboron compound that is liquid at room temperature are used, polymer particles having a smaller average particle diameter are advantageous in that they exert higher thickening effect when they are mixed with the organoboron compound, the organoboron compound that is liquid at room temperature can be easily pasted even if the particles are added in small amounts, and the resulting paste is excellent in properties, storage stability and discharge properties from a discharge nozzle of a container. Further, as the size of the particles is decreased, the influence on the progress of polymerization reaction becomes smaller when the resulting polymerization reaction initiator paste composition is mixed with a monomer. Moreover, mixing with the monomer or dispersing becomes easier, and therefore, the influence on the adhesion properties of the polymerizate becomes smaller.

On the other hand, polymer particles having a relatively larger diameter exert lower thickening effect when they are mixed with the organoboron compound, so that they can be added to the liquid organoboron compound in larger amounts than the particles of smaller diameters. As a result, the proportion of the boron compound in the polymerization initiator paste composition can be decreased. Accordingly, if the variability of the discharge amount (that is proportional to the length of the paste discharged) of the polymerization initiator paste composition is intended to be decreased, or if the amount of the polymerization initiator paste composition is intended to be increased, or if the proportion of the organoboron compound in the polymerization initiator paste composition is intended to be adjusted, particles having a large average particle diameter are used singly or in combination with particles having a small average particle diameter, whereby the prescribed intention can be attained.

In the case where the polymer particles are used as the particles (B) in the invention, particles soluble in the organoboron compound that is liquid at 25°C and particles insoluble therein can be properly selected and used in combination with the proviso that those particles are inert to the organoboron compound.

That is to say, in a preferred embodiment, polymer particles insoluble in the organoboron compound that is liquid at 25°C are added in large amounts and polymer particles soluble therein and/or inorganic particles are added in small amounts.

Further, two or more kinds of polymer particles which are insoluble but dispersible in the liquid organoboron compound and/or inorganic particles may be used in combination.

In this case, it is preferable to use, as the particles (B), mixtures of particles (b1) having an average particle diameter of not less than 0.001 µm and less than 1 µm and particles (b2) having an average particle diameter of 1 µm to 50 µm, because discharge properties of the paste from a discharge nozzle having a small inner diameter become better than the case of using only the particles (b2). The mixing ratio can be arbitrarily selected according to the purpose, and for the purpose of addition of large amounts of the polymer particles, the particles (b1) are used in amounts of 5 to 200 parts by weight, preferably 5 to 100 parts by weight, based on 100 parts by weight of the particles (b2).

As the polymer particles, any of crosslinked particles and uncrosslinked particles are properly employed.

The polymer particles may be soluble or insoluble in the polymerizable monomer component of the adhesive unless they have an influence on the adhesion strength of the adhesive, but the polymer particles soluble in the liquid organoboron compound are preferably soluble also in the polymerizable monomer.

On the other hand, the polymer particles insoluble in the liquid organoboron compound may be soluble or insoluble in the polymerizable monomer unless they have an influence on the adhesion strength.

The inorganic particles having an average particle diameter of 0.001 to 50 µm for use in the invention may be spherical or in unfixed shapes, and the shape and the particle diameter of the particles are properly selected. As the types of the inorganic particles, publicly known ones are available. For example, there can be mentioned Group I elements, Group II elements, Group III elements, Group IV elements and transition metals of the periodic table, their oxides, hydroxides, chlorides, sulfates, sulfites, carbonates, phosphates and silicates, mixtures thereof and composite salts thereof. More specifically, inorganic particles unreactive to the boron compound are properly selected from glass powders, such as silicon dioxide, strontium glass, lanthanum glass and barium glass, quartz powder, barium sulfate, aluminum oxide, titanium oxide, barium salt, calcium carbonate, glass bead, glass fiber, barium fluoride, lead salt, glass filler containing talc, colloidal silica, silica gel, zirconium oxide, tin oxide and other ceramic powders.

These inorganic particles may be used in combination of two or more kinds, and may be used together with the aforesaid polymer particles.

From the viewpoints of adhesion properties and paste properties, the average particle diameter of the inorganic particles is more preferably 0.001 to 10 µm, most preferably not less than 0.005 µm and less than 1 µm.

Similarly to the polymer particles, by the use of mixtures of inorganic fine particles (b3) having an average particle diameter of not less than 0.001 µm and less than 1 µm and inorganic particles (b4) having an average particle diameter of 1 µm to 50 µm, discharge properties of the polymerization initiator paste composition from a discharge nozzle having a small inner diameter become better than the case of using only the inorganic fine particles (b3) having an average particle diameter of not less than 0.001 µm and less than 1 µm. When the particles having different average particle diameters are used in combination as above, the mixing ratio therebetween can be arbitrarily selected according to the purpose. For the purpose of addition of large amounts of the inorganic particles, the inorganic fine particles (b3) are used in amounts of 5 to 200 parts by weight, preferably 5 to 100 parts by weight, based on 100 parts by weight of the inorganic particles (b4).

In the polymerization initiator paste composition of the invention, the particles (B) are contained in amounts of 4 to 400 parts by weight, preferably 5 to 300 parts by weight, particularly preferably 5 to 200 parts by weight, based on 100 parts by weight of the organoboron compound (A).

The polymerization initiator paste composition of the invention comprising the organoboron compound (A) and the particles (B) has a consistency, as measured at 25°C in accordance with a method defined in ISO4823, of 15 to 100 mm. Adjustment of a consistency of the polymerization initiator paste composition of the invention in the above range makes it possible to fill the polymerization initiator paste composition of the invention in a container such as a tube or a syringe and to extrude (or squeeze) the paste composition from a nozzle of the container.

The consistency of the composition of the invention is measured by the following method that is defined in ISO4823. On a glass plate, 0.5 ml of the composition is placed at 25°C using a syringe having an opening diameter of 10 mm, and on the composition, another glass plate was further placed. Then, a weight is gently placed so that the total weight of the glass plate placed on the composition and the weight should be 300±3 g, and after 1 minute, the weight is removed. Then, many pairs of parallel lines in contact with an outer edge of the shape of the composition having spread on the glass plate are drawn, and the maximum value and the minimum value between the parallel lines are measured. The measured values is calculated and expressed in the unit of 1 mm as the consistency.

Accordingly, a large value of the consistency means that the paste has characteristics that it is liable to run out and can be easily mixed with the polymerizable monomer but the composition extruded from the container cannot retain its shape and is liable to run out. Therefore, the shape of the extruded paste readily changes, and as a result, errors in the quantitative relation between the length of the paste extruded and the amount of the organoboron compound are liable to occur. On the contrary, a small value of the consistency means that the paste has a high viscosity and the shape of the composition extruded from the container is retained. In this case, errors in the quantitative relation between the length of the paste extruded and the amount of the organoboron compound hardly occur, but it becomes difficult to extrude the composition from the container, and blending properties of the composition with the polymerizable monomer are lowered.

The balance between them is extremely improved by adjusting the consistency of the paste of the invention in the range of 20 to 90 mm, particularly preferably 20 to 80 mm.

The polymerization initiator paste composition of the invention comprises the specific organoboron compound
(A) and the specific particles (B) and has a consistency in the specific range, as described above. To the polymerization initiator paste composition, however, other components can be added within limits not detrimental to the properties of the paste composition.

For example, a polymerization initiator of photopolymerization type may be further added to the polymerization initiator paste composition of the invention.

The polymerization initiator of photopolymerization type (photopolymerization initiator) is preferably one capable of undergoing photopolymerization when irradiated with visible light. The photopolymerization initiator is not specifically restricted provided that it is unreactive to the boron compound, and examples of such compounds include α-diketone compounds such as benzil, diacetyl, d,l-camphorquinone, acylphosphine oxide, ketal compounds, and thioxanthone compounds.

The polymerization initiator of photopolymerization type is used in an amount of usually 0.1 to 50 parts by weight, preferably 1 to 30 parts by weight, more preferably 1 to 20 parts by weight, based on 100 parts by weight of the organoboron compound (A).

The polymerization initiator paste composition of the invention can be used in combination with a reducing compound. As the reducing compound, an organic reducing compound is preferably used. Examples of the organic reducing compounds include aromatic amines, such as N,N-dimethylaniline, N,N-dimethyl-p-toluidine (DMPT), N,N-diethyl-p-toluidine, N,N-diethanol-p-toluidine (DEPT), N,N-dimethyl-p-tert-butylaniline, N,N-dimethylanisidine, N,N-dimethyl-p-chloroaniline, N,N-dimethylaminobenzoic acid and its alkyl esters, N,N-diethylaminobenzoic acid (DEABA) and its alkyl esters, and N,N-dimethylaminobenzaldehyde (DMABAd); N,N-dimethylaminoethyl (meth)acryalte, N,N-diethylaminoethyl (meth)acrylate, N-phenylglycine (NPG), N-tolylglycine (NTG), and N,N-(3-methacryloyloxy-2-hydroxypropyl)phenylglycine (NPG-GMA).

Of these, DMPT, DEPT, DEABA, DMABAd, NPG and NTG can be preferably used.

The organic reducing compound is used in an amount of usually 0.1 to 20 parts by weight, preferably 0.5 to 10 parts by weight, more preferably 1 to 5 parts by weight, based on 100 parts by weight of the organoboron compound (A).

The photopolymerization initiator and the reducing compound may be directly added to the polymerization initiator paste composition, or may be used by impregnating a sponge or a microbrush with them and mixing them with the polymerization initiator paste composition immediately before the paste composition is used.

In the polymerization initiator paste composition of the invention, a non-protonic solvent may be further contained. The non-protonic solvent has a function of controlling the consistency of the paste as a solvent for the organoboron compound (A) and the particles (B), particularly the polymer particles, and has a function of inhibiting fuming and firing when the liquid organoboron compound (A) is brought into contact with a combustible substance.

As the non-protonic solvent, a solvent having a boiling point of usually 30 to 150°C; preferably 30 to 120°C, particularly preferably 30 to 80°C, is employed.

The non-protonic solvent is preferably a solvent which evaporates or scatters for a short period of time after it is used and does not remain in the cured composition so as not to lower the adhesion properties of the adhesive.

As such a non-protonic solvent, a solvent having no active hydrogen of a hydroxyl group, a mercapto group or the like that reacts with the liquid organoboron compound (A) and capable of dissolving or homogeneously dispersing the liquid organoboron compound (A) to form a homogeneous solution (or dispersion) is preferably employed.

Examples of the non-protonic solvents employable in the invention include hydrocarbons, such as pentane, hexane, cyclohexane, heptane, benzene and toluene; halogenated hydrocarbons, such as chlorobenzene, fluorobenzene, dichloroethane and so-called Flon™; ethers, such as diethyl ether, diisopropyl ether, ethylene glycol dimethyl ether and tetrahydrofuran; ketones, such as acetone, methyl ethyl ketone and diethyl ketone; and esters, such as methyl acetate, ethyl acetate and isopropyl acetate. Of these, ketones, ethers and esters are preferable, and acetone, methyl ethyl ketone and ethyl acetate are particularly preferable. These non-protonic solvents may be used singly or in combination of two or more kinds.

The non-protonic solvent is used in an amount of usually 10 to 150 parts by weight, preferably 20 to 100 parts by weight, based on 100 parts by weight of the organoboron compound (A).

The organoboron compound (A) which is liquid at 25°C, the particles (B) which are inert to the organoboron compound and are solid at 25°C, and if necessary, other components can be mixed by a mixing apparatus usually used for preparing a paste, such as a stirring mixer, a static mixer or a rotating or revolving type stirring defoaming machine, in an inert gas atmosphere, preferably in a nitrogen gas atmosphere.

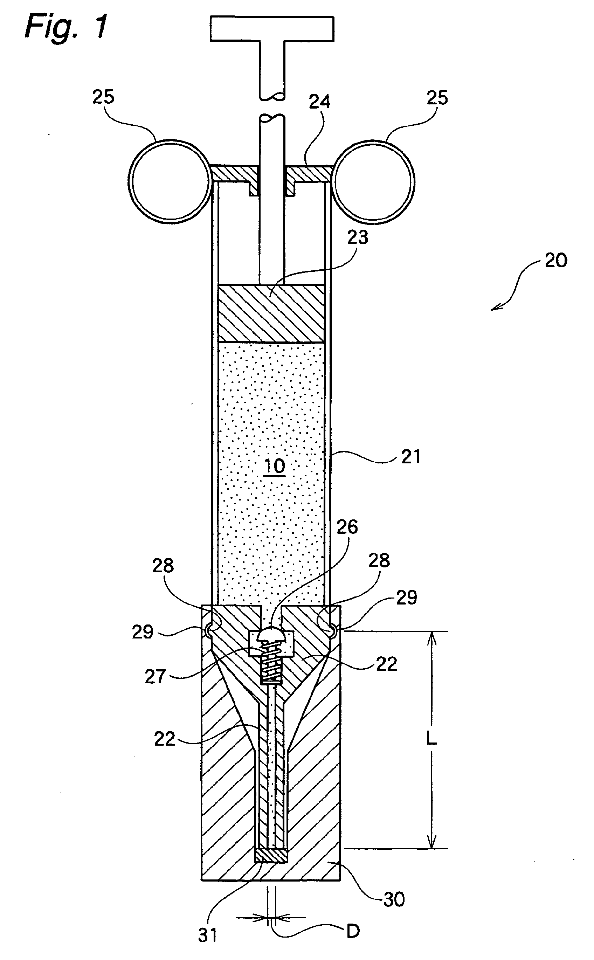

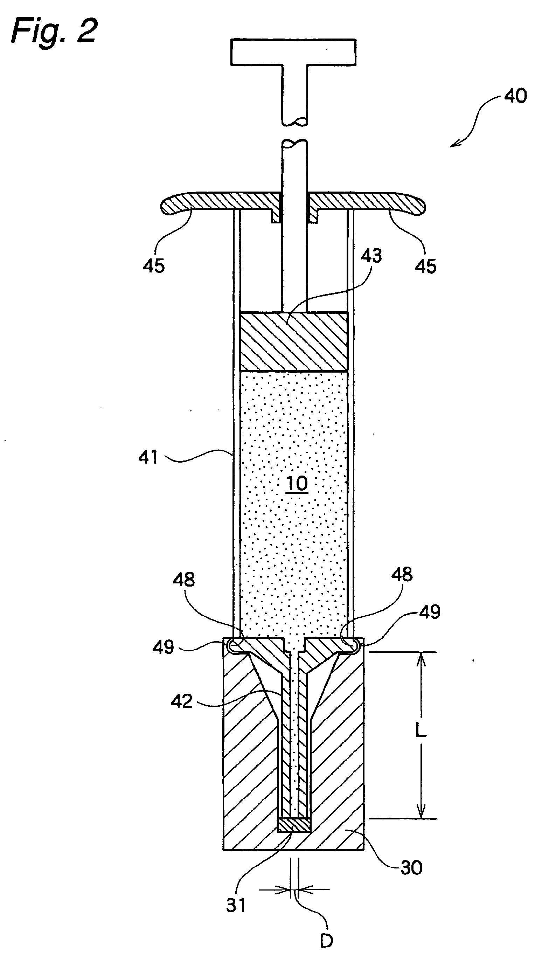

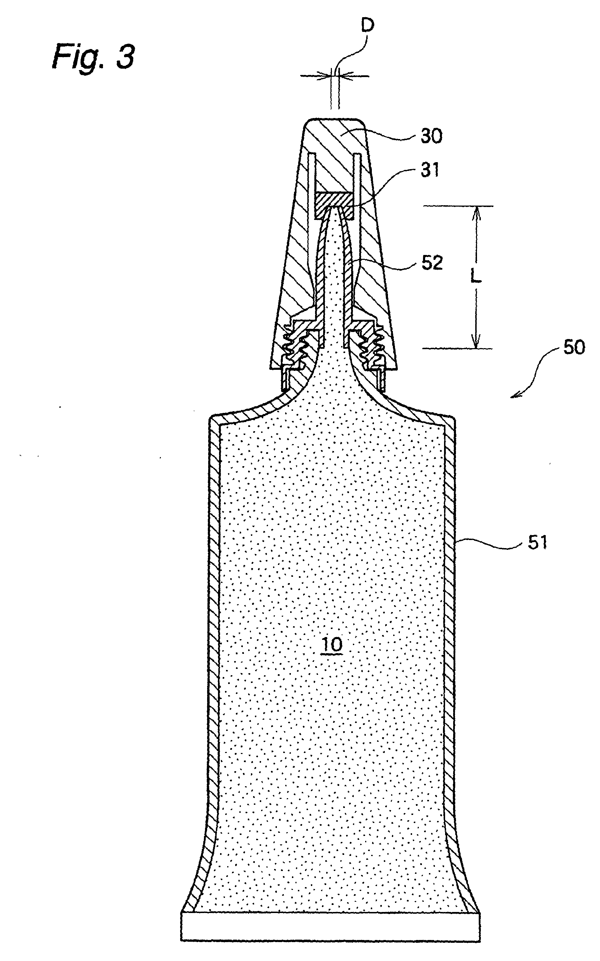

The polymerization initiator paste composition prepared by, for example, the above process is filled in a container made of a material impermeable to air and water vapor and inert to the organoboron compound, such as metal, plastic or glass, for example, a syringe 20 or 40 shown in Fig. 1 or Fig. 2 or a tube 50 shown in Fig. 3, or a packaging bag made of a laminated film of an aluminum foil and a plastic film. Then, the container or the bag is sealed. When the polymerization initiator paste composition contains a photopolymerization catalyst, a light-screening container is employed.

Examples of the metal materials employable for forming the container to be filled with the polymerization initiator paste composition of the invention include metals, such as aluminum, tin, lead, brass and stainless steel, and composite metals, such as nickel-plated brass.

Examples of the plastics preferably used for forming the container to be filled with the polymerization initiator paste composition of the invention include polyolefin resins, such as polyethylene and polypropylene, and fluororesins, such as polytetrafluoroethylene (PTFE), polyperfluoroalkyl vinyl ether (PFA), a tetrafluoroethylene/hexafluoroethylene copolymer (FEP), an ethylene fluoride/propylene fluoride ether copolymer (EPE) and an ethylene/tetrafluoroethylene copolymer (ETFE).

As the material of the tube, a metal such as aluminum or tin is preferably used singly. A laminated tube formed from a laminate of the metal and a plastic is also preferably used.

In Fig. 1, an example of a syringe filled with the polymerization initiator paste composition 10 of the invention is shown. The syringe 20 has a cylinder 21 in a cylindrical form that is filled with the polymerization initiator paste composition 10, a nozzle 22 provided at the tip of the cylinder 21 and a piston 23 to push out the polymerization initiator paste composition 10 filled in the cylinder. The cylinder 21 is in a cylindrical form so that the piston 23 can be forced thereinto. The cylinder 21 preferably has light-screening properties in order to maintain the thus filled polymerization initiator paste composition 10 stable. It is preferable that the rear end of the cylinder 21 is sealed with a sealing lid 24 having at the center an insertion hole for the piston 23. In the syringe 20 shown in Fig. 1, the rear end of the cylinder 21 is provided with a finger grip 25 in the form of a ring for laying a finger to push the piston 23. At the tip of the cylinder 21, a nozzle 22 is formed, and in the nozzle 22, a back-flow valve 26 to prevent back flow of the polymerization initiator paste composition 10 from the tip of the nozzle 22 is preferably arranged. When the back-flow valve 26 is arranged, the back-flow valve 26 is usually powered in the direction of the rear end of the cylinder 21 by means of an elastic member 27. The nozzle 22 is fixed to the tip of the cylinder 21 and has an inner diameter D of such a degree that the polymerization initiator paste composition 10 having the above consistency can be extruded. Further, the nozzle 22 has a given length L so that the polymerization initiator paste composition 10 filled in the cylinder 21 should not be influenced by air or water vapor entering from the tip of the nozzle.

It is preferable to arrange a cap 30 on the nozzle 22 so that air or the like hardly invades from the tip of the nozzle 22. The cap 30 is preferably provided with a rubber hold-down member 31 at the position that is brought into contact with the tip of the nozzle 22, and when the syringe is not used, the tip of the nozzle 22 is held down by the rubber hold-down member 31 to prevent contact of the polymerization initiator paste composition 10 present at the tip of the nozzle 22 with air or the like. It is also preferable that the cap 30 is formed so as to be united with the syringe 20 by means of, for example, a circular groove 29 which can be fitted to a circular protrusion 28 formed on the outer periphery of the cylinder 21. The cap 30 may be formed so as to be screwed into the syringe 20.

The polymerization initiator paste composition 10 of the invention can be filled in such a simple syringe 40 as shown in Fig. 2 instead of the above-mentioned syringe.

Similarly to the syringe 20 shown in Fig. 1, the syringe 40 containing the polymerization initiator paste composition 10, which is shown in Fig. 2, has a cylinder 41 in a cylindrical form that is filled with the polymerization initiator paste composition 10, a nozzle 42 provided at the tip of the cylinder 41 and a piston 43 to push out the polymerization initiator paste composition 10 filled in the cylinder.

The cylinder 41 and the piston 43 of the syringe 40 have the same constitutions as those of the syringe 20 shown in Fig. 1. Also the syringe 40 is provided with a finger grip 45, but the finger grip 45 is not in the form of a ring.

The nozzle 42 to constitute the syringe 40 is shorter than the nozzle to constitute the syringe shown in Fig. 1, and in the nozzle 42, a back-flow valve is not provided. The nozzle 42, however, has a given length L and has an inner diameter D of a given value so that the polymerization initiator paste composition 10 can be discharged in a given length and in a necessary amount.

It is preferable to arrange a cap 30 on the nozzle 42 so that air or the like hardly invades from the tip of the nozzle 42. The cap 30 is preferably provided with a rubber hold-down member 31 at the position that is brought into contact with the tip of the nozzle 42, and when the syringe is not used, the tip of the nozzle 42 is held down by the rubber hold-down member 31 to prevent contact of the polymerization initiator paste composition 10 present at the tip of the nozzle 42 with air or the like. It is also preferable that the cap 30 is formed so as to be united with the syringe 40 by means of, for example, a circular groove 49 which can be fitted to a circular protrusion 48 formed on the outer periphery of the cylinder 41. The cap 30 may be formed so as to be screwed into the syringe 40.

The polymerization initiator paste composition 10 of the invention may be filled in such a tube 50 as shown in Fig. 3.

The tube 50 has a tube body 51 and a nozzle 52, and the nozzle 52 is formed so as to be screwed into the top of the tube body 51. Into the nozzle 52, a cap 30 is screwed, whereby the polymerization initiator paste composition 10 present at the tip of the nozzle 52 can be prevented from contact with air or water vapor.

The tube body 51 of the tube 50 is usually formed from a light-screening material, and is formed from a material impermeable to air, moisture and the like.

The nozzle 52 connected to the tube 51 has an inner diameter D of such a degree that the polymerization initiator paste composition 10 having the aforesaid consistency can be extruded in a given length and in a necessary amount, and has a given length L so that the polymerization initiator paste composition 10 filled in the tube body 51 should not be influenced by air, water vapor or the like entering from the tip of the nozzle 52 and that the discharge operation can be carried out easily.

In Fig. 3, an embodiment wherein a back-flow valve is not arranged in the nozzle 52 is shown, but such a back-flow valve and such an elastic member as shown in Fig. 1 may be arranged.

It is preferable to arrange a cap 30 on the nozzle 52 so that air or the like hardly invades from the tip of the nozzle 52. The cap 30 is preferably provided with a rubber hold-down member 31 at the position that is brought into contact with the tip of the nozzle 52, and when the tube is not used, the tip of the nozzle 52 is held down by the rubber hold-down member 31 to prevent contact of the polymerization initiator paste composition 10 present at the tip of the nozzle 52 with air or the like. The cap 30 is screwed onto the outer periphery of the nozzle 52 to ensure the contact of the tip of the nozzle 52 with the rubber hold-down member 31 of the cap 30.

In case of dental use, the amount of the polymerization initiator paste composition of the invention used one time is usually in the range of 10 to 20 mg.

In the container such as a syringe or a tube, the polymerization initiator paste composition of the invention is introduced in an amount of usually 0.5 to 5 g as the amount corresponding to several tens times. The structure or the material of the container needs to be impermeable to oxygen, water vapor, and if necessary, light. When the polymerization initiator paste composition is used as a curing material of a dental adhesive, the amount of the curing material has an influence on the pot life, curing time and adhesion properties, so that it is necessary to measure a prescribed amount accurately to a certain extent. For example, by allowing the amount of the polymerization initiator extruded and the length of the polymerization initiator extruded to have a correlation with each other, a metering method of extremely good operating properties can be obtained.

The amount of a partial oxide of the organoboron compound such as a partial oxide of TBB used one time for dental purpose is usually about 0.01 g. Therefore, in order to obtain a discharge length of about 0.5 to 2 cm of the'polymerization initiator paste corresponding to this weight, the inner diameter D of the tip of the nozzle of the syringe 20 or 40 or the tube 50 is in the range of preferably 0.4 to 4 mm, more preferably 0.8 to 3 mm, for the accurate metering.

In case of usual handling or storage, the length L of the discharge nozzle of the container has only to be such a length that the polymerization initiator composition filled in the tube or the syringe is not influenced by oxygen or water vapor. If the discharge nozzle is too long, it becomes difficult to extrude the polymerization initiator composition of high viscosity. The length L of the nozzle is in the range of usually 2 to 50 mm, preferably 5 to 20 mm, more preferably 5 to 15 mm.

In order that the polymerization initiator composition can be easily extruded from such a fine nozzle and that the extruded composition has a fixed size or weight irrespective of the extrusion method, it is very important to control the consistency of the polymerization initiator composition.

It is preferable to provide a cap 30 on the tip of the nozzle of the syringe 20 shown in Fig. 1 or the syringe 40 shown in Fig. 2 or the tube 50 shown in Fig. 3 in order to seal the nozzle when the paste composition is not used or is stored. By virtue of the cap 30, contact of the polymerization initiator paste composition of the invention with air or water vapor can be minimized. The cap 30 can be formed from a resin, a metal or the like which is inert to the organoboron compound similarly to the container. At the nozzle contact portion of the cap 30, a rubber hold-down member 31 made of an elastic material such as a fluororubber or a silicone rubber is provided to prevent contact of the polymerization initiator paste composition of the invention with air or water vapor.

The polymerization initiator paste composition of the invention containing the oraganoboron compound (A), the particles (B), and optionally, other components has the following properties: (1) In usual handling of the paste composition, the composition does not cause burning or firing of paper even if it is in contact with paper; (2) the composition is pasty; and (3) the composition is activated by oxygen in air to exhibit high polymerization activity.

The polymerization initiator paste composition can be used for a dental or surgical adhesive kit.

That is to say, there is provided by the present invention a dental or surgical adhesive comprising (a) a polymerizable monomer in an amount of 20 to 90 parts by weight, (b) a filler in an amount of 0 to 70 parts by weight and (c) the polymerization initiator paste composition of the invention in an amount of 1 to 30 parts by weight, with the proviso that the total amount of the components (a), (b) and (c) is 100 parts by weight.

Further, there is also provided by the present invention a dental or surgical paste adhesive of two-agent type comprising at least a paste monomer composition, which comprises (a) a polymerizable monomer in an amount of 30 to 95 parts by weight and (b) a filler in an amount of 5 to 70 parts by weight, with the proviso that the total amount of the components (a) and (b) is 100 parts by weight, and (c) the polymerization initiator paste composition of the invention.

Furthermore, there is also provided by the present invention a dental or surgical adhesive kit comprising at least a container containing (a) a polymerizable monomer in an amount of 20 to 90 parts by weight, a container containing (c) the polymerization initiator paste composition of the invention in an amount of 1 to 30 parts by weight, and optionally, a container containing (b) a filler in an amount of 0 to 70 parts by weight, with the proviso that the total amount of the components (a), (b) and (c) is 100 parts by weight.

Moreover, there is also provided by the present invention a dental or surgical paste adhesive kit of two-agent type comprising at least a container containing a paste monomer composition comprising (a) a polymerizable monomer in an amount of 30 to 95 parts by weight and (b) a filler in an amount of 5 to 70 parts by weight, with the proviso that the total amount of the components (a) and (b) is 100 parts by weight, and a container containing (c) the polymerization initiator paste composition of the invention.

To the dental or surgical paste adhesive kit of two-agent type, a container containing a filler, e.g., polymer particles such as PMMA, may be further attached. The filler has functions of improvement and adjustment of curing properties, adhesion properties and color tone of the adhesive.

Although the paste monomer and the polymerization initiator paste composition may be mixed in an arbitrary ratio, it is preferable from the viewpoint of metering operation that the lengths of those pastes extruded are made the same as each other.

That is to say, by making both of the monomer and the polymerization initiator composition pasty, their quantities for use can be measured, not in weight, but in length of the pastes extruded.

As the container, the syringe 20 or 40 shown in Fig. 1 or Fig. 2 or the tube 50 shown in Fig. 3 is employable.

The filler (b) for use in the dental or surgical adhesive of the invention is properly selected from an organic filler (b-1), an inorganic filler (b-2) and an inorganic-organic composite filler (b-3).

The organic filler (b-1) is, for example, a filler of a powder polymer obtained by pulverization of a polymer or polymerization. As the polymer for forming the organic filler, a homopolymer of the aforesaid polymerizable monomer or a copolymer thereof is preferably employed. Examples of such polymers include alkyl (meth)acrylate polymers, such as polymethyl methacrylate (PMMA), polypropyl methacrylate, polybutyl methacrylate (PBMA), poly(ethylene glycol dimethacrylate) and poly(trimethylolpropane trimethacrylate). As other polymers, there can be mentioned polyvinyl acetate, polystyrene, their copolymers, and butadiene copolymers such as MBS.

In the present invention, an organic filler having an average particle diameter of usually 0.005 mµ to 50 µm, preferably 0.01 mµ to 35 µm, is employed.

Examples of the inorganic fillers (b-2) include silica, silica alumina, alumina, alumina quartz, glass (including barium glass), titania (titanium oxide), zirconia (zirconium oxide), silica zirconia, calcium carbonate, kaolin, clay, mica, aluminum sulfate, barium sulfate, calcium carbonate, calcium sulfate and calcium phosphate. Such inorganic fillers may be those having been surface treated with coupling agents such as silane coupling agents.

In the present invention, an inorganic filler having an average particle diameter of usually 0.005 µm to 50 µm, preferably 0.01 µm to 20 µm, is employed.

Examples of the organic-inorganic composite fillers (b-3) include fillers obtained by mixing inorganic fillers with polymerizable monomers to perform polymerization and then pulverizing the resulting polymers. Specifically, there can be mentioned, for example, a filler obtained by polymerization coating of a silica fine powder or zirconium oxide as the inorganic filler with a polymerizable monomer mainly containing trimethylolpropane trimethacrylate and pulverizing the resulting polymer.

In the present invention, an organic-inorganic composite filler having an average particle diameter of usually 0.01 µm to 50 µm, preferably 0.5 µm to 25 µm, is employed.

As the polymerizable monomer (a) for the dental or surgical adhesive of the invention, publicly known monofunctional monomers or polyfunctional monomers can be employed without any restriction. Of such monomers, (meth)acrylate monomers are preferably employed because they are relatively lowly irritant to human bodies. Further, a polymerizable monomer having an acid group in its molecule is preferable as a component capable of particularly imparting high adhesion to dentin to the adhesive. Therefore, a combination of (meth)acrylate and a polymerizable monomer having an acid group, such as carboxyl group, its anhydride group, phosphoric acid group or sulfonic acid group, is also preferably employed.

Examples of the monofunctional (meth)acrylates employable in the invention include alkyl (meth)acrylates, such as methyl (meth)acrylate, ethyl (meth)acrylate, propyl (meth)acrylate, butyl (meth)acrylate, hexyl (meth)acrylate, 2-ethylhexyl (meth)acrylate, dodecyl (meth)acrylate, lauryl (meth)acrylate, cyclohexyl (meth)acrylate, benzyl (meth)acrylate and isobornyl (meth)acrylate; hydroxyalkyl esters of (meth)acrylic acids, such as 2-hydroxyethyl (meth)acrylate, 2- or 3-hydroxypropyl (meth)acrylate, 4-hydroxybutyl (meth)acrylate, 5-hydroxypentyl (meth)acrylate, 6-hydroxyhexyl (meth)acrylate, 1,2- or 1,3-dihydroxypropyl mono(meth)acrylate and erythritol mono(meth)acrylate; polyethylene glycol mono(meth)acrylates, such as diethylene glycol mono(meth)acrylate, triethylene glycol mono(meth)acrylate, polyethylene glycol mono(meth)acrylate and polypropylene glycol mono(meth)acrylate; (poly)glycol monoalkyl ether (meth)acrylates, such as ethylene glycol monomethyl ether (meth)acrylate, ethylene glycol monoethyl ether (meth)acrylate, diethylene glycol monomethyl ether (meth)acrylate, triethylene glycol monomethyl ether (meth)acrylate, polyethylene glycol monomethyl ether (meth)acrylate and polypropylene glycol monoalkyl ether (meth)acrylate; fluoroalkyl esters of (meth)acrylic acids, such as perfluorooctyl (meth)acrylate and hexafluorobutyl (meth)acrylate; silane compounds having (meth)acryloxyalkyl groups, such as γ-(meth)acryloxypropyltrimethoxysilane and γ-(meth)acryloxypropyltri(trimethylsiloxy)silane; and (meth)acrylates having heterocyclic rings, such as tetrafurfuryl (meth)acrylate.

Examples of the polyfunctional (meth)acrylates include:
poly(meth)acrylates of alkanepolyols, such as ethylene glycol di(meth)acrylate, propylene glycol di(meth)acrylate, butylene glycol di(meth)acrylate, neopentyl glycol di(meth)acrylate, hexylene glycol di(meth)acrylate, trimethylolpropane tri(meth)acrylate and pentaerythritol tetra(meth)acryalte;
polyoxyalkanepolyol poly(meth)acrylates, such as diethylene glycol di(meth)acrylate, triethylene glycol di(meth)acrylate, polyethylene glycol di(meth)acrylate, dipropylene glycol di(meth)acrylate, polypropylene glycol di(meth)acrylate, dibutylene glycol di(meth)acrylate and dipentaerythritol hexa(meth)acrylate;
aliphatic or aromatic di(meth)acrylates represented by the following formula (1): 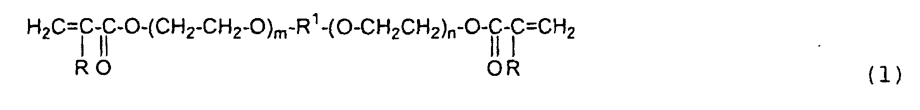 wherein R is a hydrogen atom or a methyl group, m and n are the same or different and are each a number of 0 to 10, and R¹ is any one of 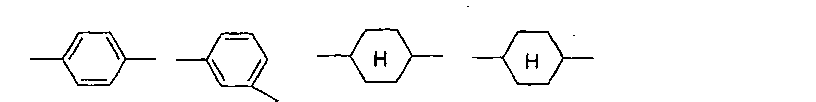 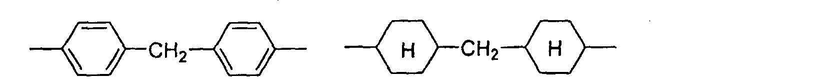 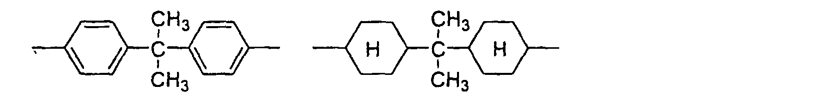 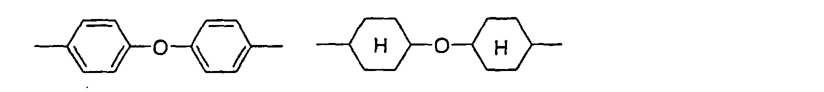 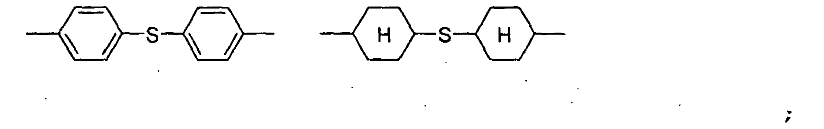
aliphatic or aromatic epoxy di(meth)acrylates represented by the following formula (2): 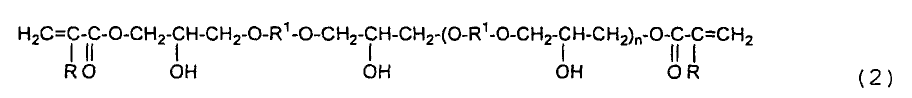 wherein R is a hydrogen atom or a methyl group, n is a number of 0 to 10, and R¹ is any one of 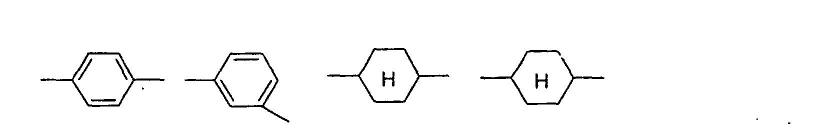 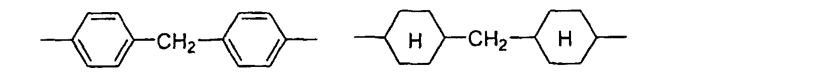 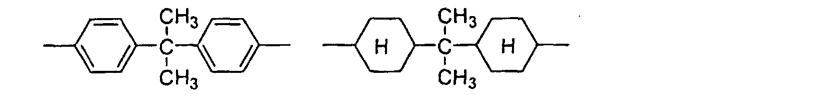 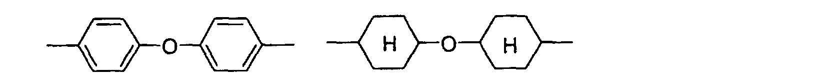 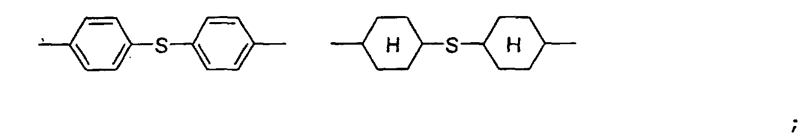 and
polyfunctional (meth)acrylates having a urethane bond in a molecule, which are represented by the following formula (3): 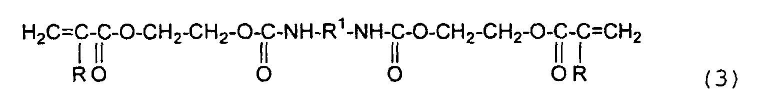 wherein R is a hydrogen atom or a methyl group, and R¹ is any one of

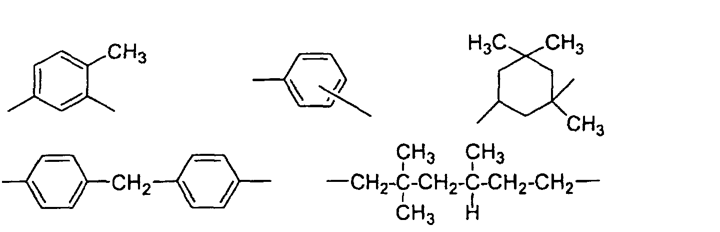

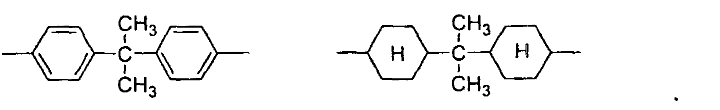

Of the above monofunctional (meth)acrylates, particularly preferably used are alkyl (meth)acrylates, such as methyl (meth)acrylate and ethyl (meth)acrylate; (meth)acrylates having hydroxyl group, such as 2-hydroxyethyl (meth)acrylate, 1,3-dihydroxypropyl mono(meth)acrylate and erythritol mono(meth)acrylate; and (meth)acrylates having ethylene glycol chain in a molecule, such as triethylene glycol mono methyl ether (meth)acrylate and triethylene glycol mono(meth)acrylate.

Of the above polyfunctional (meth)acrylates, particularly preferably used are di(meth)acrylates having ethylene glycol chain in a molecule, such as triethylene glycol di(meth)acryalte and polyethylene glycol di(meth)acrylate;
compounds represented by the following formula (1)-a: 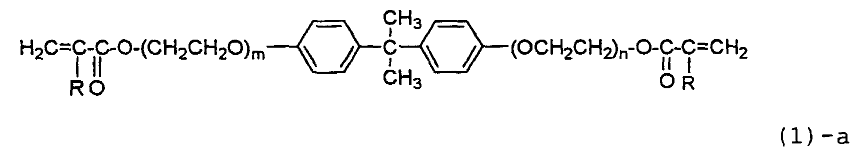 wherein R, m and n have the same meanings as in the formula (1);
compounds represented by the following formula (2)-a: 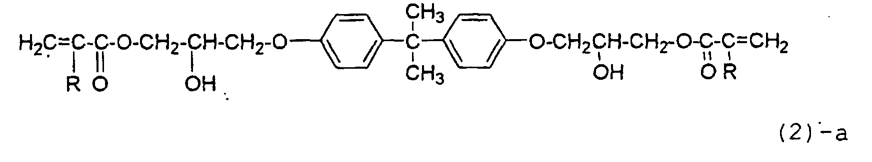 wherein R has the same meaning as in the formula (2); and
compounds represented by the following formula (3)-a: 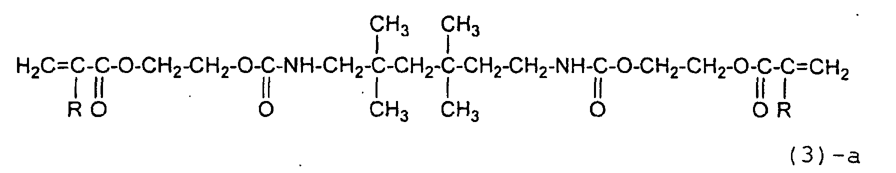 wherein R has the same meaning as in the formula (3).

The above monomers can be used singly or in combination of two or more kinds.

Examples of the polymerizable monomers having an acid group in a molecule include (meth)acrylic acid ester monomers having carboxylic acid group or carboxylic acid anhydride group, such as (meth)acrylic acid or its anhydride, 1,4-di(meth)acryloxyethylpyromellitic acid, 6-(meth)acryloxyethylnaphthalene-1,2,6-tricarboxlyic acid, N-(meth)acryloyl-p-aminobenzoic acid, N-(meth)acryloyl-o-aminobenzoic acid, N-(meth)acryloyl-m-aminobenzoic acid, N-(meth)acryloyl-5-aminosalicylic acid, N-(meth)acryloyl-4-aminosalicylic acid, 4-(meth)acryloxyethyltrimellitic acid or its anhydride, 4-(meth)acryloxybutyltrimellitic acid or its anhydride, 4-(meth)acryloxyhexyltrimellitic acid or its anhydride, 4-(meth)acryloxydecyltrimellitic acid or its anhydride, 2-(meth)acryloyloxybenzoic acid, 3-(meth)acryloyloxybenzoic acid, 4-(meth)acryloyloxybenzoic acid, β-(meth)acryloyloxyethyl hydrogensuccinate, β-(meth)acryloyloxyethyl hydrogenmaleate, β-(meth)acryloyloxyethyl hydrogenphthalate, 11-(meth)acryloyloxy-1,1-undecanedicarboxylic acid and p-vinylbenzoic acid; (meth)acrylic acid ester monomers having phosphoric acid group, such as (2-(meth)acryloxyethyl)phosphoric acid, (2-(meth)acryloxyethylphenyl)phosphoric acid and 10-(meth)acryloxydecylphosphoric acid; and monomers having sulfonic acid group, such as p-styrenesulfonic acid and 2-acrylamido-2-methylpropanesulfonic acid.

The above monomers having an acid group can be used singly or in combination. It is preferable to use the polymerizable monomer having an acid group in an amount of 2 to 20 parts by weight based on 100 parts by weight of the total of the polymerizable monomers (a).

Examples of the (meth)acrylate polymers (b) contained in the adhesive of the invention include non-crosslinking polymers, such as polymethyl (meth)acrylate, polyethyl (meth)acryalte, a methyl (meth)acryalte/ethyl (meth)acrylate copolymer, a methyl (meth)acrylate/butyl (meth)acrylate copolymer and a methyl (meth)acrylate/styrene copolymer; and crosslinking polymers, such as a methyl (meth)acrylate/ethylene glycol di(meth)acrylate copolymer, a methyl (meth)acrylate/triethylene glycol di(meth)acrylate copolymer and a copolymer of methyl (meth)acrylate and a butadiene type monomer.

It is also possible to use inorganic particles wherein metal oxides or metal salts are coated with the above alkyl methacrylate polymers. The component (b) or the component (c) may be used after mixed with the component (a).

In the adhesive of the invention, the component (b) is used in an amount of 0 to 70 parts by weight and the component (c) is used in an amount of 3 to 25 parts by weight, based on 20 to 90 parts by weight of the component (a), with the proviso that the total of the components (a), (b) and (c) is 100 parts by weight.

To the adhesive of the invention, camphorquinone, organic solvents, such as acetone, coloring agents, and polymerization inhibitors, such as hydroquinones, can be added in appropriate amounts.

The adhesive of the invention is applied to dental or surgical use. When the adhesive of the invention is applied to dental or surgical use, it is preferable to use it after pretreatment of teeth. Examples of the pretreatments include an etching treatment of an adhesive surface with an acid solution, a modification treatment of an adhesive surface with a primer, and an etching and modification treatment of an adhesive surface with a primer having etching ability. Examples of the acid solutions used for the etching treatment include a phosphoric acid aqueous solution having a concentration of 5 to 60% by weight and an aqueous solution containing 10% by weight of citric acid and 3% by weight of ferric chloride. The primer used for the modification treatment of an adhesive surface is, for example, an aqueous solution of a concentration of 20 to 50% by weight containing 2-hydroxyethyl (methyl) acrylate and 1,3-dihydroxypropyl mono(meth)acrylate. As the primer having etching ability used for the etching and modification treatment of an adhesive surface, an aqueous solution containing an organic acid (including a monomer having an acid group) and a component to modify decalcificated dentin and thereby accelerate diffusion of the adhesive into the dentin is preferably employed. Examples of the components to accelerate diffusion of the adhesive into dentin include monomers having hydroxyl group, such as alkylene glycol, polyalkylene glycol, 2-hydroxyethyl (meth)acrylate and 1,3-dihydroxypropyl mono(meth)acrylate, and polyethylene glycol (meth)acryalte.

The polymerization initiator paste composition of the invention and the adhesive using the paste composition have good biocompatibility and are preferably used for adhesive restoration of tissues, e.g., adhesive restoration of teeth, protection of wounded hard or soft tissues or adhesive fixation in surgical treatment.

### INDUSTRIAL APPLICABILITY

The polymerization initiator paste composition of the invention is controlled to have a given consistency by adding specific particles to an organoboron compound that is liquid at room temperature, and therefore, it becomes possible to fill the paste composition in a container such as a tube or a syringe and to extrude a necessary amount of the paste composition from the container when the paste composition is used. Further, the amount of the paste composition used can be measured, not in weight, but in length of the extruded paste composition. Moreover, because the polymerization initiator paste composition of the invention has the above-mentioned consistency, it can be easily extruded from a container such as a syringe, and the shape of the extruded polymerization initiator paste composition is not broken and can be retained as it is. Accordingly, from the length of the extruded polymerization initiator paste composition, the amount of the polymerization initiator can be accurately measured.

The dental or surgical adhesive of the invention comprises the polymerization initiator paste composition and a polymerizable monomer that is packaged separately from the paste composition, and the amount of the polymerization initiator can be accurately measured in length. Further, by making both the components pasty, mixing of those components becomes very easy.

In the dental or surgical adhesive kit of the invention, the polymerization initiator composition is a paste, and therefore, the composition can be quantitatively determined and used by measuring the composition in length. Moreover, mixing of the composition with a polymerizable monomer is very easy.

### EXAMPLE

The present invention is further described with reference to the following examples, but it should be construed that the invention is in no way limited to those examples.

### Measuring methods

### Preparation of polymerization initiator paste composition containing organoboron compound

(1) In a nitrogen atmosphere, given amounts of a filler and an organoboron compound (including partially oxidized organoboron compound) were mixed by a spatula to prepare a polymerization initiator paste composition. The paste compositions of Examples 10 and 11 further contained acetone in an amount of 15% by weight and 13% by weight, respectively.
(2) The polymerization initiator paste composition obtained in the above step (1) was filled in a syringe having a diameter of 10 mm in a nitrogen atmosphere, and the syringe was placed in an aluminum coating multilayer bag with a zipper and stored in a nitrogen box.
(3) In the preparation of an adhesive, a given length of each component was extruded by the use of a syringe with a discharge opening having an inner diameter of 0.9 mm or 1.8 mm, and the components were mixed.

### Measurement of consistency

The consistency was measured in accordance with a method described in ISO4823. That is to say, at 25°C, 0.5 ml of the composition was extruded from a syringe (opening: 10 mm) having a diameter of 10 mm and placed on a surface of a first glass plate. On the composition, a second glass plate and a weight (total: 300g±3g) were gently placed, and after 1 minute, the weight was removed. Then, many pairs of parallel lines in contact with an outer edge of the shape of the composition having spread on the glass plate were drawn, and the maximum value and the minimum value between the parallel lines were measured. An average of the measured values was calculated and expressed in the unit of 1 mm.

### Examples 1 - 11

Tributylboron (oxygen addition quantity: 0.8% by mol) and a filler shown in Table 1 were mixed in the same manner as described above to prepare a polymerization initiator paste composition.

The polymerization initiator paste composition was filled in a commercially available 2.5 ml plastic syringe and discharged from a discharge nozzle (inner diameter: 0.9 mm or 1.8 mm, length: 10 mm), followed by examination of discharge properties of the polymerization initiator paste composition.

In Table 1, AA means that the composition was well discharged; BB means that the composition was badly discharged; and CC means that the composition was not discharged.

Then, adhesion strength of the polymerization initiator paste composition to dentin was measured in the following manner.
(1) Under water pouring, a lip side surface of a front tooth of cattle was cut to expose dentin, and the dentin was polished with emery paper of No. 600 to form an adhesive surface.
   The adhesive surface was dried, then treated with an etching solution containing 10% by weight of citric acid and 3% by weight of iron chloride for 10 seconds, washed with water for 10 seconds and then subjected to air blow drying for 15 seconds. Thereafter, a cellophane tape having a circular hole of 4 mm diameter was attached to the adhesive surface to specify the adhesion area.
(2) On a Dappen dish, 4 drops (0.32 g) of a polymerizable monomer consisting of MMA/4-META=95/5 (parts by weight) and the polymerization initiator paste composition in such an amount that the amount of TBBO became about 0.07 g were placed, and they were lightly mixed for 10 seconds to give a resin mud.
(3) The resin mud was applied onto the adhesive surface prepared in the above step (1), and an acrylic bar was bonded to prepare an adhesion test sample. The adhesion test sample was allowed to stand for 30 minutes at room temperature, immersed in distilled water at 37°C for 24 hours and then subjected to a tensile test to measure adhesion strength between the acrylic bar and the dentin. The adhesion strength is an average of measured values of three test samples.

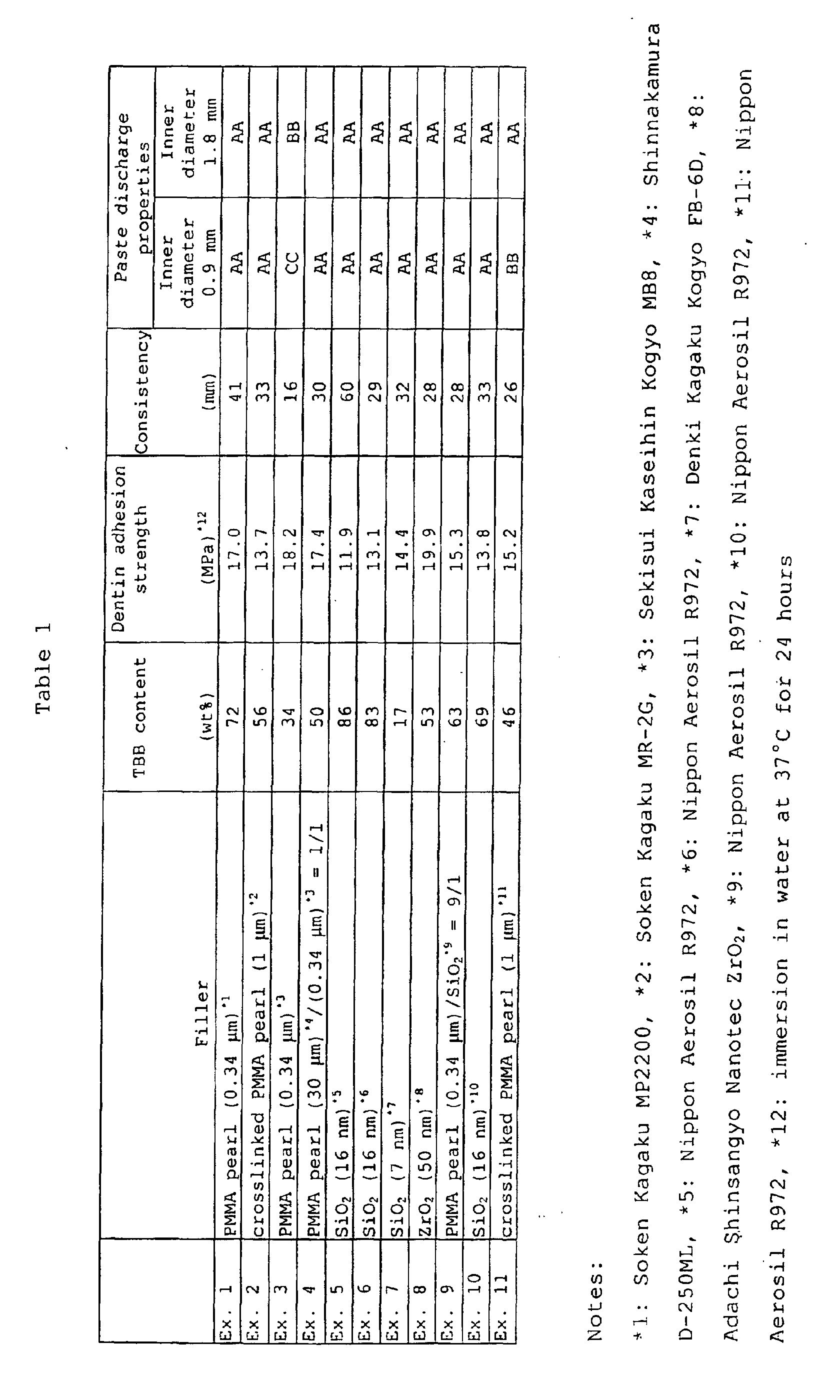

## Claims

1. A polymerization initiator paste composition comprising:
(A) an organoboron compound, and
(B) particles having an average particle diameter of 0.001 to 50 µm and inert to the organoboron compound (A), in an amount of 4 to 400 parts by weight based on 100 parts by weight of the organoboron compound (A),
said paste composition having a consistency, as measured at 25°C under a load of 300 g in accordance with a method defined in ISO4823, of 15 to 100 mm.

2. The polymerization initiator paste composition as claimed in claim 1, wherein the organoboron compound (A) is liquid at 25°C.

3. The polymerization initiator paste composition as claimed in claim 1, wherein the organoboron compound (A) is trialkylboron or a partial oxide thereof.

4. The polymerization initiator paste composition as claimed in claim 1, wherein the particles (B) are polymer particles which are inert to the organoboron compound (A) and are insoluble but homogeneously dispersible in the organoboron compound (A).

5. The polymerization initiator paste composition as claimed in claim 1, wherein the particles (B) are metal oxide particles having an average particle diameter of 0.001 to 10 µm and inert to the organoboron compound (A).

6. The polymerization initiator paste composition as claimed in claim 1, wherein the particles (B) are mixtures of fine particles (b1) having an average particle diameter of not less than 0.001 µm and less then 1 µm and particles (b2) having an average particle diameter of 1 µm to 50 µm.

7. The polymerization initiator paste composition as claimed in claim 1, which is contained in a sealed container having impermeability to air and water vapor and having a nozzle that is capable of extruding the polymerization initiator paste composition and has a nozzle tip inner diameter of 0.4 to 4 mm and a nozzle length of 2 to 50 mm.

8. The polymerization initiator paste composition as claimed in claim 7, wherein the container having impermeability to air and water vapor is a tube or a syringe.

9. A dental or surgical adhesive comprising:
a polymerization initiator paste composition which comprises:
(A) an organoboron compound, and
(B) particles having an average particle diameter of 0.001 to 50 µm and inert to the organoboron compound (A), in an amount of 4 to 400 parts by weight based on 100 parts by weight of the organoboron compound (A), and
has a consistency, as measured at 25°C under a load of 300 g in accordance with a method defined in ISO4823, of 15 to 100 mm,
and
(C) a polymerizable monomer which is packaged separately from the polymerization initiator paste composition.

10. The dental or surgical adhesive as claimed in claim 9, wherein the organoboron compound (A) is trialkylboron or a partial oxide thereof.

11. A dental or surgical adhesive kit comprising:
a first container filled with a polymerization initiator paste composition which comprises:
(A) an organoboron compound, and
(B) particles having an average particle diameter of 0.001 to 50 µm and inert to the organoboron compound (A), in an amount of 4 to 400 parts by weight based on 100 parts by weight of the organoboron compound (A),
has a consistency, as measured at 25°C under a load of 300 g in accordance with a method defined in ISO4823, of 15 to 100 mm, and
is extrudable from the container,
and
a second container filled with a polymerizable monomer (C) which is contained separately from the polymerization initiator paste composition.

12. The dental or surgical adhesive kit as claimed in claim 11, wherein the first container can contain the polymerization initiator paste composition without substantially bringing the paste composition into contact with air and water vapor and has a nozzle capable of extruding the polymerization initiator paste composition and having a nozzle tip inner diameter of 0.4 to 4 mm and a nozzle length of 2 to 50 mm.

13. The dental or surgical adhesive kit as claimed in claim 12, wherein the nozzle tip inner diameter of the first container is in the range of 0.5 to 3 mm.

14. The dental or surgical adhesive kit as claimed in claim 11, wherein the organoboron compound (A) is trialkylboron or a partial oxide thereof.

15. The dental or surgical adhesive kit as claimed in claim 11, wherein the polymerizable monomer has a consistency equal to that of the polymerization initiator paste composition.
